# EUROPÄISCHE PATENTSCHRIFT

(11) **EP 3 461 435 B1**
(45) Veröffentlichungstag und Bekanntmachung des Hinweises auf die Patenterteilung: **04.09.2019**
(21) Anmeldenummer: 17193622.2
(22) Anmeldetag: 28.09.2017
(51) Int. Cl.: A61B 17/122, A61B 17/00

(54) **CHIRURGISCHER CLIP AUS KARBONFASERVERSTÄRKTEM KUNSTSTOFFMATERIAL**
SURGICAL CLIP MADE FROM CARBON FIBRE REINFORCED PLASTIC MATERIAL
PINCE CHIRURGICALE EN MATÉRIAU PLASTIQUE RENFORCÉ PAR DES FIBRES EN CARBONE

(43) Veröffentlichungstag der Anmeldung: 03.04.2019
(73) Patentinhaber: Lazic Besitz GmbH & Co. KG, 78532 Tuttlingen (DE)
(72) Erfinder: Lazic, Daniel, 78532 Tuttlingen (DE)
(74) Vertreter: Kohler Schmid Möbus Patentanwälte

(56) Entgegenhaltungen:
- WO-A1-2016/107952
- DE-A1-102009 003 273
- DE-A1-102013 107 876
- DE-C2- 19 935 418
- ALEXANDER BRACK ET AL: "Development of an artifact-free aneurysm clip", CURRENT DIRECTIONS IN BIOMEDICAL ENGINEERING, Bd. 2, Nr. 1, 30. September 2016 (2016-09-30), Seiten 543-546, XP055423361, DOI: 10.1515/cdbme-2016-0120

## Beschreibung

Die Erfindung betrifft einen chirurgischen Clip, insbesondere Aneurysma-Clip, mit zwei drehbar miteinander verbundenen Clipteilen und mit einer Schenkelfeder, deren zwei Federschenkel jeweils an den beiden Clipteilen abgestützt sind, um die beiden Clipteile gegeneinander vorzuspannen, wobei die Schenkelfeder aus einem mit Karbonendlosfasern verstärkten Kunststoffmaterial, insbesondere PEEK, gebildet ist und die Karbonendlosfasern entlang der Federwindungen der Schenkelfeder ausgerichtet sind.

Ein derartiger chirurgischer Clip ist beispielsweise durch den Artikel von Alexander Brack et al., "Development of an artifact-free aneurysm clip", CURRENT DIRECTIONS IN BIOMEDICAL ENGINEERING, Bd. 2, Nr. 1, 30. September 2016 (2016-09-30), Seiten 543-546, bekannt geworden. Dieser bekannte Clip weist zwei Clipteile aus Kunststoff und eine Schraubenfeder aus Kunststoff auf, wobei der Zusammenbau des Clips durch Kunststoffschweißen der Schraubenfeder mit den Clipteilen erfolgt.

Aus DE 10 2009 003 273 A1 oder DE 10 2004 016 859 A1 sind chirurgische Clips bekannt, die zwei miteinander verbundene Clipteile aus Metall und eine Schenkelfeder aus Metall umfassen, deren Federschenkel mit den Clipteilen verschweißt sind.

Aus DE 10 2013 107876 A1 ist weiterhin ein chirurgischer Clip mit einem ersten Klemmarm und einem zweiten Klemmarm bekannt, die drehbar miteinander gekoppelt und mittels einer Feder in einer Drehrichtung gegeneinander in eine geschlossene Stellung vorgespannt sind. An dem ersten Klemmarm ist ein erstes Gehäuseteil und an dem zweiten Klemmarm ein zweites Gehäuseteil ausgebildet oder angeordnet, welche in Kooperation ein Gehäuse ausbilden, in dem die Schraubenfeder aufgenommen ist. Das eine, innere Federende ist an einem Achsstift befestigt, der eine Drehachse definiert, um die die beiden Gehäuseteile relativ zueinander schwenkbar sind. Das andere äußere Federende ist an einem radial nach innen vorstehenden Vorsprung eines der der beiden Gehäuseteile abgestützt.

Aus DE 199 35 418 A1 ist schließlich noch ein chirurgischer Clip mit zwei relativ zueinander um eine Drehachse verschwenkbaren Klemmarmen bekannt, die in einer Klemmstellung einen aneinander anliegenden Klemmbereich ausbilden und jeweils ein freies und ein mit einem Lager versehenes Ende aufweisen. In den beiden Lagern ist eine gemeinsame, die Drehachse definierende Schraubenfeder gelagert. Die Schraubenfeder ist den beiden Klemmarmen zugeordnet und hält diese in der Klemmstellung unter Vorspannung. Dazu ist das eine Federende in einer Ausnehmung des einen Klemmarms und das andere Federende außen an dem anderen Klemmarm abgestützt.

Demgegenüber liegt der vorliegenden Erfindung die Aufgabe zugrunde, bei einem chirurgischen Clip der eingangs genannten Art die Abstützung bzw. Befestigung der Schenkelfeder an den Clipteilen zu verbessern.

Diese Aufgabe wird erfindungsgemäß dadurch gelöst, dass die beiden Clipteile jeweils einen in Richtung der Drehachse der beiden Clipteile axial vorstehenden Vorsprung aufweisen, an dem der jeweilige Federschenkel der Schenkelfeder abgestützt ist.

Die Schenkelfeder ist aus einem röntgentransparenten Faserverbundmaterial hergestellt, das aus PEEK-Material, welches als Implantatmaterial zugelassen ist, und Karbonendlosfasern besteht. Die Karbonendlosfasern sind entlang der Federwindungen ausgerichtet, um der Schenkelfeder bei Biegebeanspruchung ausreichende Stabilität zu verleihen.

Besonders bevorzugt sind auch die beiden Clipteile aus einem röntgentransparenten Kunststoffmaterial, insbesondere PEEK (Polyetheretherketon), gebildet. Dabei können die beiden Clipteile vorteilhaft aus einem mit Karbonkurzfasern verstärkten Kunststoffmaterial, insbesondere PEEK, gebildet sein, also aus einem röntgentransparenten Faserverbundmaterial, in dem die Karbonkurzfasern im Gefüge ungeordnet mit dem Peek verbunden sind. In dieser Ausführung ist der gesamte Clip aus röntgentransparentem Material gebildet.

Um die Schenkelfeder an den beiden Clipteilen abzustützen, können die beiden Federschenkel mit den beiden Clipteilen stoffschlüssig verbunden, insbesondere miteinander (ultraschall)verschweißt sein. Der Vorsprung kann hakenförmig ausgebildet sein, um den jeweiligen Federschenkel radial zu übergreifen und dadurch axial festzulegen, oder der jeweilige Federschenkel kann in dem Vorsprung zumindest teilweise formschlüssig eingebettet, insbesondere eingeschmolzen sein.

Vorzugsweise weisen die beiden Clipteile jeweils einen Klemmarm, einen Bedienarm und einen dazwischen befindlichen Drehlagerabschnitt mit einer Öffnung auf, wobei die Drehlagerabschnitte der beiden Clipteile drehbar ineinander gelagert sind und die Öffnungen der beiden Clipteile eine axiale Durchgangsöffnung ausbilden, in der zumindest einige der Federwindungen der Schenkelfeder angeordnet sind.

Weitere Vorteile und vorteilhafte Ausgestaltungen des Gegenstands der Erfindung ergeben sich aus der Beschreibung, den Ansprüchen und den Zeichnungen. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung. Die vorliegende Erfindung wird durch die Merkmale des unabhängigen Anspruchs definiert. Bevorzugte Ausführungsformen sind in den abhängigen Ansprüchen angegeben. Es zeigen:
- Fig. 1: einen chirurgischen Clip mit zwei Clipteilen und einer Schenkelfeder in seiner geschlossenen Endstellung;
- Fign. 2a, 2b: ein erstes Clipteil des in Fig.1 gezeigten Clips in einer Draufsicht (Fig. 2a) und in einer Seitenansicht (Fig. 2b);
- Fign. 3a, 3b: ein zweites Clipteil des in Fig.1 gezeigten Clips in einer Draufsicht (Fig. 3a) und in einer Seitenansicht (Fig. 3b);
- Fign. 4a-4e: verschiedene Modifikationen eines an den Clipteilen jeweils vorhandenen Vorsprungs zur Abstützung der Schenkelfeder und;
- Fign. 5a, 5b: ein weiteres Ausführungsbeispiel eines ersten oder zweiten Clip teils mit einem rampenförmigen Vorsprung in einer perspektivischen Ansicht (Fig. 5a) und in einer abgebrochenen Seitenansicht (Fig. 5b).

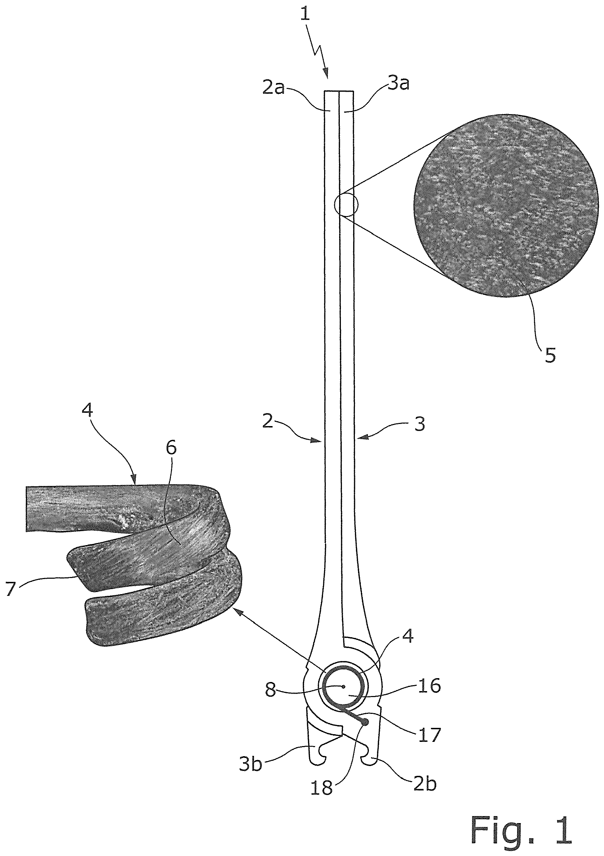

Der in **Fig. 1** gezeigte chirurgische Clip **1** umfasst zwei drehbar miteinander verbundene, scherenförmige Clipteile **2**, **3** und eine Schenkelfeder **4**, um die beiden Clipteile 2, 3 gegeneinander in die in Fig. 1 gezeigte geschlossene Endstellung vorzuspannen.

Die beiden Clipteile 2, 3 sind aus einem mit Karbonkurzfasern **5** verstärkten PEEK (Polyetheretherketon) und die Schenkelfeder 4 aus einem mit Karbonendlosfasern **6** verstärkten PEEK gebildet. Der Clip 1 ist somit aus einem röntgentransparenten Faserverbundmaterial hergestellt, das aus Karbonfasern 5, 6 und PEEK-Material, welches als Implantatmaterial zugelassen ist, besteht. Im Gegensatz zu den beiden Clipteilen 2, 3, in denen die Karbonkurzfasern 5 im Gefüge ungeordnet mit dem Peek verbunden sind, sind bei der Schenkelfeder 4 die Karbonendlosfasern 6 entlang den Federwindungen **7** ausgerichtet, um der Schenkelfeder 4 bei Biegebeanspruchung ausreichende Stabilität zu verleihen.

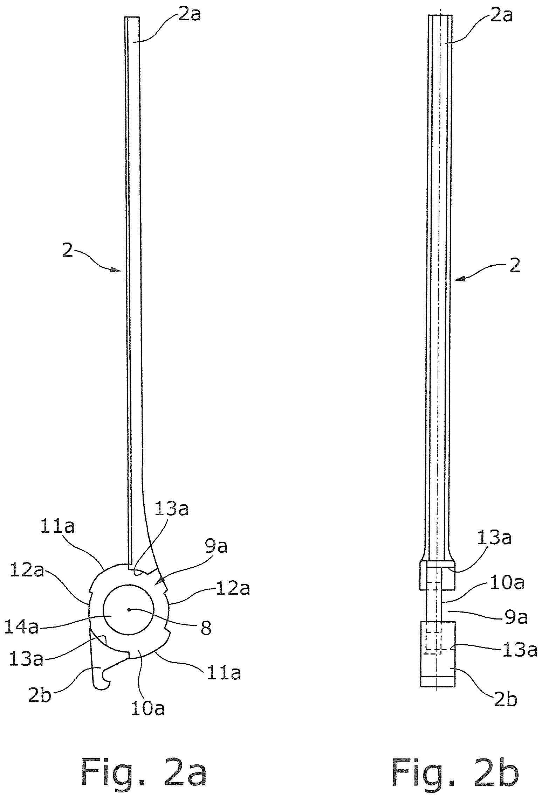

Wie in **Fign. 2a, 2b** gezeigt, weist das eine, erste Clipteil 2 einen Klemmarm **2a** und einen Bedienarm **2b**, die bezüglich der Drehachse **8** einander gegenüberliegen, sowie dazwischen einen Drehlagerabschnitt **9a** auf. Der Drehlagerabschnitt 9a ist als axial offene Steckaufnahme ausgebildet, deren Boden bzw. Bodenplatte **10a** ringförmig ausgebildet ist und am Außenumfang zwei einander gegenüberliegende, äußere Drehführungsabschnitte **11a** und dazwischen jeweils eine radial nach innen zurückspringende Montageaussparung **12a** aufweist. Am Übergang von der Bodenplatte 10a zum Klemmarm 2a bzw. zum Bedienarm 2b sind zwei bezüglich der Drehachse 8 einander gegenüberliegende, innere Drehführungsabschnitte **13a** vorgesehen, die der Drehachse 8 zugewandt und jeweils durch einen Absatz am Klemmarm 2a und am Bedienarm 2b gebildet sind. In der Bodenplatte 10a ist eine zur Drehachse 8 zentrische Öffnung **14a** vorgesehen.

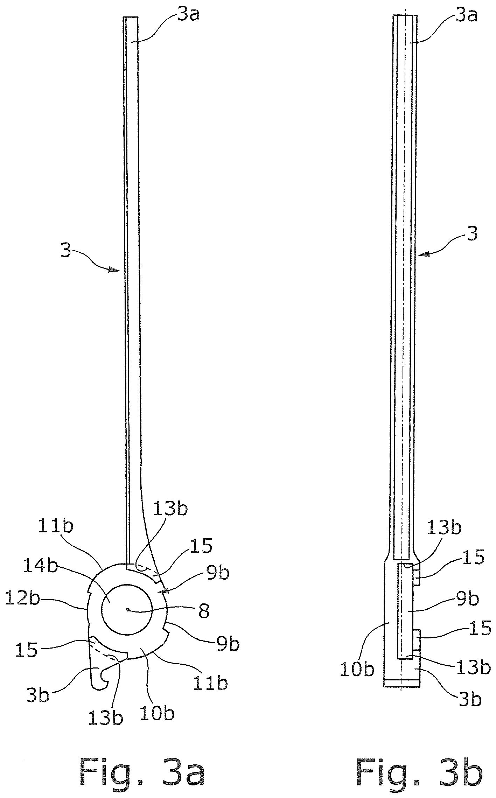

Wie in **Fign. 3a, 3b** gezeigt, weist das andere, zweite Clipteil 3 einen Klemmarm **3a** und einen Bedienarm **3b**, die bezüglich der Drehachse 8 einander gegenüberliegen, sowie dazwischen einen Drehlagerabschnitt **9b** auf. Der Drehlagerabschnitt 9b ist als axial offene Steckaufnahme ausgebildet, deren Boden bzw. Bodenplatte **10b** ringförmig ausgebildet ist und am Außenumfang zwei einander gegenüberliegende, äußere Drehführungsabschnitte **11b** und dazwischen jeweils eine radial nach innen zurückspringende Montageaussparung **12b** aufweist. Am Übergang von der Bodenplatte 10b zum Klemmarm 3a bzw. zum Bedienarm 3b sind zwei bezüglich der Drehachse 8 einander gegenüberliegende, innere Drehführungsabschnitte **13b** vorgesehen, die der Drehachse 8 zugewandt und jeweils durch einen Absatz am Klemmarm 3a und am Bedienarm 3b gebildet sind. In der Bodenplatte 10b ist eine zur Drehachse 8 zentrische Öffnung **14b** vorgesehen. Die inneren Drehführungsabschnitte 13b sind auf der der Bodenplatte 10b gegenüberliegenden Seite jeweils von einem Überstand **15** übergriffen und dadurch als Führungsnut ausgebildet. Mit Ausnahme seiner beiden Überstände 15 ist das zweite Clipteil 3 identisch dem ersten Clipteil 2 ausgebildet.

Zum Verbinden der beiden Clipteile 2, 3 zum chirurgische Clip 1 werden die Clipteile 2, 3 mit ihren beiden Montageaussparungen 12a bzw. 12b jeweils zwischen den beiden äußere Drehführungsabschnitte 11b bzw. 11a des jeweils anderen Clipteils ausgerichtet und axial ineinander gesteckt, bis sie mit ihren Bodenplatten 10a, 10b aneinander anliegen. Anschließend werden die beiden Clipteile 2, 3 in Richtung auf die in Fig. 1 gezeigte geschlossene Endstellung verdreht, wodurch sowohl die äußeren Drehführungsabschnitte 11a des ersten Clipteils 2 in die Führungsnuten 13b des zweiten Clipteils 3 eingreifen und darin drehbar gelagert sind als auch die äußeren Drehführungsabschnitte11b des zweiten Clipteils 3 an den inneren Drehlagerabschnitten 13a des ersten Clipteils 2 drehbar gelagert sind. Durch den Eingriff der äußeren Drehführungsabschnitte 11a in die Führungsnuten 13b sind die beiden Clipteile 2, 3 miteinander axial verbunden bzw. entgegen der Steckrichtung verriegelt.

Die zentrischen Öffnung 14a, 14b der miteinander verbundenen Clipteile 2, 3 bilden eine Durchgangsöffnung **16**, in der die Schenkelfeder 4 mit ihren Federwindungen 7 angeordnet ist. Die beiden Federschenkel **17** der Schenkelfeder 4 sind bei **18** mit den Bodenplatten 10a, 10b der beiden Clipteilen 2, 3 durch Ultraschallschweißen stoffschlüssig verbunden und dadurch an den beiden Clipteilen 2, 3 abgestützt. Mithilfe einer zwischen die beiden Bedienarme 2b, 3b greifenden Anlegezange können die Bedienarme 2b, 3b gegen die Kraft der Schenkelfeder 4 auseinandergedrückt und dadurch die Klemmarme 2a, 3a geöffnet werden.

Statt der in Fign. 1 bis 3 gezeigten Steck-Drehschluss-Lagerung können die beiden Clipteile 2, 3 auf jegliche andere Art und Weise zueinander unmittelbar oder mittelbar drehbar gelagert sein, also beispielsweise durch eine Nut-FederLagerung wie in Fign. 12 bis 16 der DE 10 2004 016 859 A1, durch eine Rast- oder Schnappdrehverbindung wie in DE 103 09 491 B4, durch die Schenkelfeder 4 wie in DE 199 35 418 C2 oder durch eine angeformte oder separate Lagerhülse wie in DE 10 2013 200 127 A1.

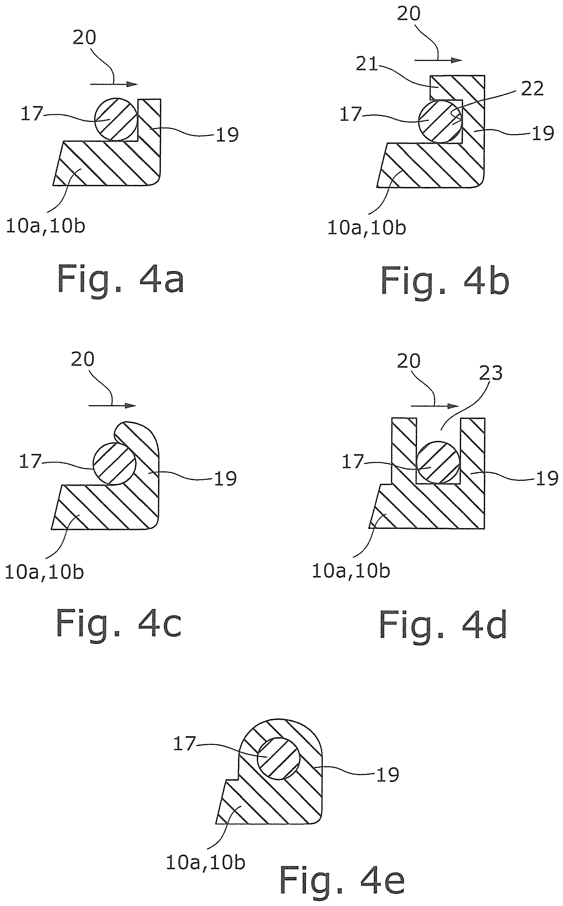

In **Fign. 4a-4e** sind verschiedene Varianten der Abstützung der beiden Federschenkel 17 an einem axial vorstehenden Vorsprung **19** der Clipteile 2, 3 gezeigt. Der Vorsprung 19 ist einstückig an den Clipteilen 2, 3 angeformt und somit ebenfalls aus mit Karbonkurzfasern 5 verstärktem PEEK gebildet. Bevorzugt ist der Vorsprung 19 außen am Drehlagerabschnitt 9a, 9b, also an der Bodenplatte 10a, 10b, angeformt, kann aber alternativ auch am Klemmarm 2a, 3a oder am Bedienarm 2b, 3b angeformt sein. Der Federschenkel 17 ist am Vorsprung 19 zumindest in Kraftrichtung **20** seiner Federwirkung abgestützt.

In Fig. 4a ist der Federschenkel 17 am Vorsprung 19 abgestützt, aber nicht axial festgelegt.

In Fig. 4b ist der Vorsprung 19 hakenförmig mit einem Überstand **21** und somit mit einer entgegen der Kraftrichtung 20 offenen Nut **22** ausgebildet, in die der Federschenkel 17 eingelegt und, weil axial übergriffen, axial festgelegt ist.

In Fig. 4c ist der Federschenkel 17 vom Vorsprung 19 teilweise formschlüssig umgriffen und dadurch axial festgelegt. Beispielsweise kann der Vorsprung 19 durch eine Wärmebehandlung um den Federschenkel 17 herum umgeschmolzen bzw. verformt worden sein.

In Fig. 4d weist der Vorsprung 19 einen axial einseitig offenen Schlitz **23** auf, in den der Federschenkel 17 eingelegt und dadurch in und entgegen der Richtung 20 seiner Federwirkung abgestützt ist.

In Fig. 4e ist der Federschenkel 17 in dem Vorsprung 19 vollständig formschlüssig eingebettet bzw. eingeschmolzen. Beispielsweise kann der Vorsprung 19 zunächst einen einseitig offenen Schlitz aufweisen, dessen offenes Schlitzende, nachdem der Federschenkel 17 in den Schlitz eingelegt worden ist, durch eine Wärmebehandlung umgeschmolzen und dadurch geschlossen worden ist.

Der in Fign.1 bis 4 gezeigte, chirurgische Clip 1 ist somit vollständig aus karbonfaserverstärktem PEEK, also aus röntgentransparentem Material, gebildet.

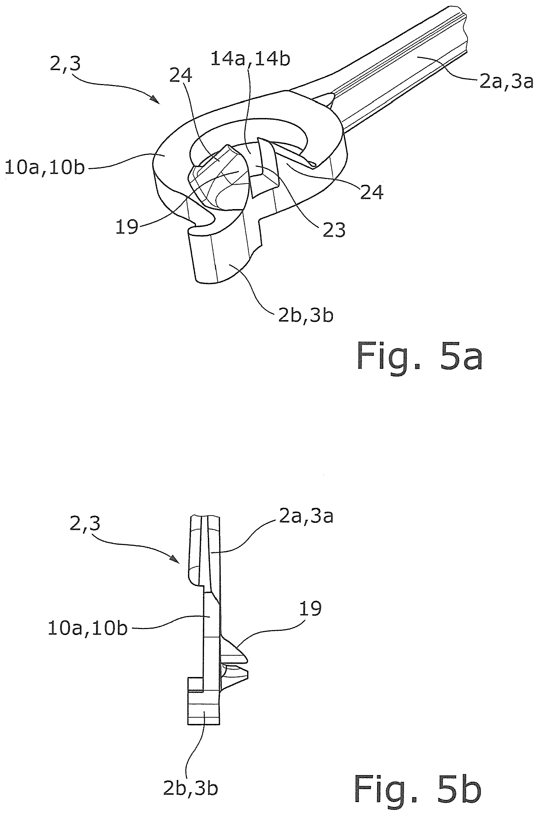

Das in **Fign. 5a, 5b** gezeigte Clipteil 2, 3 dient hier sowohl als erstes als auch als zweites Clipteil, d.h., der Clip ist aus zwei baugleichen Clipteilen 2, 3 zusammengesetzt. Der Vorsprung 19 ist wie in Fig. 4d mit einem axial einseitig offenen Schlitz 23 ausgebildet, dessen beide Seitenwände **24** jeweils als eine zum Schlitz 22 hin ansteigende Rampe ausgebildet sind.

## Patentansprüche

1. Chirurgischer Clip (1), insbesondere Aneurysma-Clip, mit zwei drehbar miteinander verbundenen Clipteilen (2, 3) und mit einer Schenkelfeder (4), deren zwei Federschenkel (17) jeweils an den beiden Clipteilen (2, 3) abgestützt sind, um die beiden Clipteile (2, 3) gegeneinander vorzuspannen, wobei die Schenkelfeder (4) aus einem mit Karbonendlosfasern (6) verstärkten Kunststoffmaterial, insbesondere PEEK, gebildet ist und die Karbonendlosfasern (6) entlang der Federwindungen (7) der Schenkelfeder (4) ausgerichtet sind,
**dadurch gekennzeichnet,**
**dass** die beiden Clipteile (2, 3) jeweils einen in Richtung der Drehachse (8) der beiden Clipteile (2, 3) axial vorstehenden Vorsprung (19) aufweisen, an dem der jeweilige Federschenkel (17) der Schenkelfeder (4) abgestützt ist.

2. Chirurgischer Clip nach Anspruch 1, **dadurch gekennzeichnet, dass** der Vorsprung (19) hakenförmig ausgebildet ist und den jeweiligen Federschenkel (17) radial übergreift.

3. Chirurgischer Clip nach Anspruch 1, **dadurch gekennzeichnet, dass** der jeweilige Federschenkel (17) in dem Vorsprung (19) zumindest teilweise formschlüssig eingebettet, insbesondere eingeschmolzen ist.

4. Chirurgischer Clip nach einem der vorhergehenden Ansprüche, **dadurch gekennzeichnet, dass** die beiden Clipteile (2, 3) aus einem Kunststoffmaterial, insbesondere PEEK, gebildet sind.

5. Chirurgischer Clip nach einem der vorhergehenden Ansprüche, **dadurch gekennzeichnet, dass** die beiden Clipteile (2, 3) aus einem mit Karbonkurzfasern (5) verstärkten Kunststoffmaterial, insbesondere PEEK, gebildet sind.

6. Chirurgischer Clip nach Anspruch 4 oder 5, **dadurch gekennzeichnet, dass** die beiden Federschenkel (17) der Schenkelfeder (4) mit den beiden Clipteilen (2, 3) stoffschlüssig verbunden, insbesondere miteinander verschweißt sind.

7. Chirurgischer Clip nach einem der vorhergehenden Ansprüche, **dadurch gekennzeichnet, dass** die beiden Clipteile (2, 3) jeweils einen Klemmarm (2a, 3a), einen Bedienarm (2b, 3b) und einen dazwischen befindlichen Drehlagerabschnitt (9a, 9b) mit einer Öffnung (14a, 14b) aufweisen, wobei die Drehlagerabschnitte (9a, 9b) der beiden Clipteile (2, 3) drehbar ineinander gelagert sind und die Öffnungen (14a, 14b) der beiden Clipteile (2, 3) eine axiale Durchgangsöffnung (16) ausbilden, in der zumindest einige der Federwindungen (7) der Schenkelfeder (4) angeordnet sind.

## Claims

1. Surgical clip (1), in particular an aneurysm clip, comprising two rotatably interconnected clip parts (2, 3) and a leg spring (4), the two spring legs (17) of which are in each case supported on the two clip parts (2, 3) so as to mutually pretension the two clip parts (2, 3),
wherein the leg spring (4) is formed from a plastics material, in particular PEEK, that is reinforced with continuous carbon fibers (6), and the continuous carbon fibers (6) are aligned along the spring coilings (7) of the leg spring (4),
**characterized in that** the two clip parts (2, 3) have in each case one protrusion (19) which axially protrudes in the direction of the axis of rotation (8) of the two clip parts (2, 3) and on which the respective spring leg (17) of the leg spring (4) is supported.

2. Surgical clip according to claim 1, **characterized in that** the protrusion (19) is configured so as to be hook-shaped and engages radially across the respective spring leg (17).

3. Surgical clip according to claim 1, **characterized in that** the respective spring leg (17) is at least partially embedded in the protrusion (19) in a form-fitting manner, in particular is melted therein.

4. Surgical clip according to any one of the preceding claims, **characterized in that** the two clip parts (2, 3) are formed from a plastics material, in particular PEEK.

5. Surgical clip according to any one of the preceding claims, **characterized in that** the two clip parts (2, 3) are formed from a plastics material, in particular PEEK, which is reinforced with short carbon fibers (5).

6. Surgical clip according to claim 4 or 5, **characterized in that** the two spring legs (17) of the leg spring (4) are connected to the two clip parts (2, 3) in a materially integral manner, in particular are welded to one another.

7. Surgical clip according to one of the preceding claims, **characterized in that** the two clip parts (2, 3) have in each case one clamping arm (2a, 3a), one operating arm (2b, 3b), and a rotary bearing portion (9a, 9b) located therebetween, said rotary bearing portion (9a, 9b) having an opening (14a, 14b), wherein the rotary bearing portions (9a, 9b) of the two clip parts (2, 3) are mounted so as to be rotatable within one another, and the openings (14a, 14b) of the two clip parts (2, 3) form an axial passage opening (16) in which at least some of the spring coilings (7) of the leg spring (4) are disposed.

## Revendications

1. Pince chirurgicale (1), notamment pince de traitement des anévrismes, comprenant deux parties de pincement (2, 3) reliées l'une à l'autre avec faculté de rotation, et un ressort (4) à branches dont les deux branches (17) sont respectivement en appui contre les deux parties de pincement (2, 3), en vue de précontraindre l'une contre l'autre lesdites deux parties de pincement (2, 3), ledit ressort (4) à branches étant constitué d'une matière plastique, en particulier de PEEK renforcé par des fibres de carbone (6) sans fin, et lesdites fibres de carbone (6) sans fin étant orientées le long des spires (7) dudit ressort (4) à branches,
**caractérisée par le fait**
**que** les deux parties de pincement (2, 3) sont respectivement munies d'une saillie (19) qui dépasse axialement dans la direction de l'axe de rotation (8) desdites deux parties de pincement (2, 3), et contre laquelle la branche considérée (17) du ressort (4) à branches est en appui.

2. Pince chirurgicale selon la revendication 1, **caractérisée par le fait que** la saillie (19) est réalisée en forme de crochet et coiffe, dans le sens radial, la branche considérée (17) du ressort.

3. Pince chirurgicale selon la revendication 1, **caractérisée par le fait que** la branche considérée (17) du ressort est au moins partiellement noyée dans la saillie (19) par complémentarité de formes, en particulier intégrée par fusion.

4. Pince chirurgicale selon l'une des revendications précédentes, **caractérisée par le fait que** les deux parties de pincement (2, 3) consistent en une matière plastique, notamment en du PEEK.

5. Pince chirurgicale selon l'une des revendications précédentes, **caractérisée par le fait que** les deux parties de pincement (2, 3) sont constituées d'une matière plastique, en particulier de PEEK renforcé par de courtes fibres de carbone (5).

6. Pince chirurgicale selon la revendication 4 ou 5, **caractérisée par le fait que** les deux branches (17) du ressort (4) à branches sont reliées matériellement aux deux parties de pincement (2, 3), notamment solidarisées par soudage.

7. Pince chirurgicale selon l'une des revendications précédentes, **caractérisée par le fait que** les deux parties de pincement (2, 3) sont respectivement dotées d'un bras de coincement (2a, 3a), d'un bras d'actionnement (2b, 3b) et d'une zone intercalaire (9a, 9b) de montage rotatif, percée d'un orifice (14a, 14b), les zones (9a, 9b) de montage rotatif des deux parties de pincement (2, 3) étant montées à rotation l'une dans l'autre, et les orifices (14a, 14b) desdites deux parties de pincement (2, 3) donnant naissance à un orifice axial de passage (16) dans lequel au moins quelques-unes des spires (7) du ressort (4) à branches sont disposées.
